# EUROPÄISCHE PATENTSCHRIFT

(11) **EP 2 542 151 B1**
(45) Veröffentlichungstag und Bekanntmachung des Hinweises auf die Patenterteilung: **06.05.2015**
(21) Anmeldenummer: 11707651.3
(22) Anmeldetag: 03.03.2011
(51) Int. Cl.: A61B 5/04, A61B 5/0402, A61B 5/20, A61M 5/14, G01N 33/493, G01N 33/68, G01N 33/487, G01N 35/00, G01N 33/49, A61M 5/142

(54) **SYSTEM ZUR ERMITTLUNG VON THERAPIEBEZOGENEN DATEN FÜR DIE VERABREICHUNG VON MEDIKAMENTEN AN ZU THERAPIERENDEN PATIENTEN UNTER BERÜCKSICHTIGUNG VON INDIVIDUELLEN WERTIGKEITSFAKTOREN**
SYSTEM FOR DETERMINING TREATMENT-RELATED DATA FOR THE ADMINISTRATION OF DRUGS TO PATIENTS TO BE TREATED TAKING INTO ACCOUNT IMPORTANT INDIVIDUAL FACTORS
SYSTÈME DE DÉTERMINATION DE DONNÉES THÉRAPEUTIQUES POUR L'ADMINISTRATION DE MÉDICAMENTS À DES PATIENTS À TRAITER AVEC PRISE EN COMPTE DE FACTEURS DE COEFFICIENT INDIVIDUELS

(30) Priorität: 24.03.2010 DE 102010012733; 05.03.2010 DE 102010010567
(43) Veröffentlichungstag der Anmeldung: 09.01.2013
(73) Patentinhaber: B. Braun Melsungen AG, 34212 Melsungen (DE)
(72) Erfinder: MAIER, Hans-Otto, 34212 Melsungen (DE); DÖNHOFF, Torsten, USA RIVER (TZ); SCHMOLL, Horst, 34302 Guxhagen (DE); PAETZOLD, Matthias, 34212 Melsungen (DE)
(74) Vertreter: Winter, Brandl, Fürniss, Hübner, Röss, Kaiser, Polte - Partnerschaft mbB
(86) Internationale Anmeldenummer: PCT/EP2011/053243
(87) Internationale Veröffentlichungsnummer: WO 2011/107566

(56) Entgegenhaltungen:
- WO-A1-2005/050497
- US-A1- 2001 047 252
- US-A1- 2007 015 972

## Beschreibung

Die Erfindung betrifft ein System zur Ermittlung von therapiebezogenen Daten für die Verabreichung von mindestens einem Medikament an einen zu therapierenden Patienten mit mindestens einer Entnahmeeinrichtung zum fortlaufenden und aufeinanderfolgenden Entnehmen von Blutmengen von dem Patienten zum Erhalten einer Mehrzahl an Blutproben, mindestens einer, vorzugsweise mehrerer Blutwertemesseinrichtungen zum Messen von Blutwerten der entnommenen Blutproben und zum Erhalten von Blutwertemessdatensätzen und mindestens eine Berechnungseinrichtung zum Errechnen therapiebezogener Daten aus den Blutwertemessdatensätzen und gegebenenfalls weiteren Datensätzen gemäß dem Oberbegriff der Patentansprüche 1 und 8.

Herkömmlicherweise werden Patienten, die insbesondere auf der Intensivstation liegen, mit Medikamenten und gegebenenfalls künstlicher Nahrung mittels einer oder mehrerer Zuführeinrichtungen, beispielsweise intravenös oder mittels Magensonde versorgt. Beispielsweise kann die Zuführeinrichtung eine Insulinzuführeinrichtung bzw. eine Infusionspumpe sein, die den innerhalb des Blutkreislaufes des Patienten vorhandenen Insulinwerte auf ein vorbestimmbares Niveau in Reaktion auf einen zuvor gemessenen Blutglukosewert im Blutkreislauf des Patienten hält. Ebenso können Zuführeinrichtungen für mindestens einen Nahrungswert einer mit mindestens einer Nahrungszuführeinrichtung dem Blutkreislauf direkt oder indirekt zugeführten Nahrung dem Patienten verabreicht werden.

Sämtliche derartige Zuführeinrichtungen, auch wenn sie in einem System zur Verabreichung von Medikamenten und/oder Nahrungswerten integriert sind, erfordern bisher die durch einen Arzt oder ein weiteres klinisches Personal zu erfolgende Eingabe von Werten, die der Zuführung mittels der Zuführeinrichtung zugrunde liegen und beispielsweise können hier Mengenwerte, Zeitabstände, in welchen die Zuführungen erfolgen soll, intervallartige Zuführungen etc. als Basis für die anschließend zu erfolgende Zuführung von beispielsweise Insulin eingegeben werden.

Derartige Eingaben von Insulinwerten und damit zusammenhängender Medikamentenparameter beruhen häufig auf Erfahrungswerten des klinischen Personals, wobei zuvor festgestellte Blutwerte hierbei berücksichtigt werden.

Dieser Zuführung vorausgehend ist eine zumeist von Hand durchgeführte Blutentnahme, bei dem Patienten, welche die Zwischenschaltung von klinischem Personal erfordert. Ebenso ist weiteres klinisches Personal erforderlich, welches die notwendigen Fachkenntnisse zu den Eingabefunktionen der Zuführeinrichtung, wie der Infusionspumpe, hat, um anschließend diese Zuführung durchzuführen.

Es ist möglich, dass bei einer Infusionspumpe die Eingabe einer Dosiseinheit ermöglicht wird. Allerdings erfordert dies die Berücksichtigung von Angaben über die Konzentration des Wirkstoffes des zu verabreichenden Medikamentes und über die Art des Medikamentes. Sowohl bei der Angabe der Konzentration des Wirkstoffes, als auch bei der Eingabe der Dosiseinheit und der Umrechnung derjenigen in die Förderrate werden bisher ausschließlich die Hauptwirkstoffe des Medikamentes berücksichtigt. Dies ist häufig ausreichend, sofern lediglich oder vorrangig ein spezifisches Medikament verabreicht werden soll. Auch in diesem Fall wird auf Erfahrungswerte und Datenlisten des klinischen Personals, insbesondere eines Arztes zurückgegriffen.

Die in diesem Zusammenhang verwendeten Infusionspumpen-Systeme werden häufig bei Patienten, die einer intensiven medizinischen Behandlung bedürfen, verwendet. Hierbei weisen die Infusionspumpen die Eigenschaft der kontinuierlichen genauen Dosierung der Medikation in ihrer Zuführung auf. Um eine optimierte Abstimmung dieser Pumpen in ihrer Dosierung zu erreichen, werden die Pumpen in einem gemeinsamen Ordnungssystem integriert, welches üblicherweise eine zentrale Steuereinheit, Bedienungseinheit und eine Alarmierungseinheit aufweist.

Zwar werden während einer Behandlung bzw. Therapie eines Patienten die ermittelten Blutwerte der vorausgegangenen Blutwertemessungen tabellarisch von Hand oder in elektronischer Form archiviert, jedoch findet eine Beurteilung der Medikamentenparameter für die aktuelle Verabreichung des Medikamentes nur dann unter Berücksichtigung der in der Vergangenheit liegenden Blutwerte statt, wenn der Arzt bzw. das klinische Personal sich veranlasst fühlt, diese Listen bzw. Tabellen einzusehen.

Somit wird bei den bisherigen Systemen und dem bisherigen Verfahren zur Berechnung von Medikamentenparametern und therapeutischen Daten für die Therapie eines Patienten routinemäßig keine Miteinbeziehung der in der Vergangenheit liegenden Messwerte praktiziert.

Benachbarter Stand der Technik ist auch aus den Druckschriften WO 2005/050497 A1 und US 2001/0047252 A1 bekannt.

Demzufolge ist es Aufgabe der Erfindung, ein System und ein Verfahren zur Ermittlung von therapiebezogenen Daten für die Verabreichung von mindestens einem Medikament an einen zu therapierenden Patienten zur Verfügung zu stellen, welches eine verbesserte Therapie des Patienten aufgrund einer verbesserten Berechnung der therapiebezogenen Daten unter Einbeziehung von Vergangenheitswerten, insbesondere bei Patienten auf der Intensivstation, ermöglicht.

Diese Aufgabe wird systemseitig durch die Merkmale des Patentanspruches 1 und verfahrensseitig durch die Merkmale des Patentanspruches 6 gelöst.

Ein wesentlicher Punkt der Erfindung liegt darin, dass bei einem System zur Ermittlung von therapiebezogenen Daten für die Verabreichung von mindestens einem Medikament an einem zu therapierenden Patienten folgende Merkmale vorliegen:
- Mindestens eine Entnahmeeinrichtung, um fortlaufend und aufeinanderfolgend Blutmengen von Patienten zum Erhalten einer Mehrzahl an Blutproben zu entnehmen;
- mindestens eine, vorzugsweise mehrere, Blutwertemesseinrichtung(en), um Blutwerte der entnommenen Blutproben zu messen und Blutwertemessdatensätze zu erhalten;
- eine Berechnungseinrichtung, um therapiebezogene Daten aus den Blutwertemessdatensätzen und gegebenenfalls weiteren Daten zu berechnen.

Jeder Blutprobe und jedem dazugehörigen Blutwertemessdatensatz ist zumindest eine patientenbezogene erste Identifikationskennung und eine zeitbezogene zweite Identifikationskennung über den Zeitpunkt der Entnahme der Blutprobe zugeordnet. Die Berechnungseinrichtung für die Berechnung der therapiebezogenen Daten ordnet mittels einer ersten Bewertungseinheit jedem Blutwertemessdatensatz mit der gleichen ersten Identifikationskennung und unterschiedlicher zweiter Identifikationskennung mindestens einen individuellen Wertigkeitsfaktor, vorzugsweise einen Zahlenfaktor, zur Kennzeichnung einer Wertigkeit der Blutwertemessdaten in dem Berechnungsvorgang zu.

Ein derartiges System kann vorteilhaft nicht nur unter Berücksichtigung der für die aktuelle Berechnung therapeutischer Daten gemessenen Blutwerte berücksichtigen, sondern berücksichtigt auch die Blutwerte der Vergangenheit. Hierfür wird dem Patienten während der Behandlungsdauer bzw. Beobachtungsdauer immer wieder Blut entnommen, um aufeinanderfolgende Blutproben zu erhalten. Die Anzahl der entnommenen Blutproben richtet sich nach dem jeweiligen Krankheitsbild. Jede Blutprobe wird gemäß einer Kennzeichnungseinrichtung mit patientenbezogenen ersten Identifikationskennungen und zeitbezogenen zweiten Identifikationskennungen gekennzeichnet. Dies kann beispielsweise dadurch geschehen, dass eine Spritze, die zur Aufnahme der Blutprobe dient, außenseitig mit einem Barcode-Label oder einem Datamatrix-Code-Label und/oder einem Transponder beklebt wird, auf den zumindest die Patientenidentifikation und ein Zeitstempel, welcher den Zeitpunkt der Blutentnahme wiedergibt, aufgebracht ist.

Jede erste und zweite Identifikationskennung wird jedoch auch in ihrer Zuordnung aufrecht erhalten, wenn in der jeweiligen Messeinrichtung ein Blutwertemessdatensatz aus den gemessenen Blutwerten der Blutprobe entsteht und dieser in Form eines Datensatzes an die Berechnungseinrichtung weitergesendet wird. Dies kann beispielsweise dadurch geschehen, dass das Barcode-Label und/oder das Datamatrix-Code-Label mittels eines Barcode-Scanners ausgelesen wird und die dabei ermittelten ersten und zweiten Identifikationskennungen den jeweils entstandenen Blutwertemessdatensatz nach Messung der Blutwerte datenelektronisch zugeordnet wird.

Durch eine derartige Zuordnung der ersten und zweiten Identifikationskennung ist sichergestellt, dass auch bei einer Mehrzahl von verwendeten Messeinrichtungen die jeweilige Blutprobe entsprechend gekennzeichnet ist und somit von den anderen Blutproben unterschieden werden kann.

Zusätzlich wird mittels einer Zuordnungseinrichtung, die mit jeder Messeinrichtung verbunden ist, jedem Blutwertemessdatensatz, wie er von einer Messeinrichtung zu der Berechnungseinrichtung gelangt, eine der jeweiligen Messeinrichtung zugeordnete messgerätebezogene dritte Identifikationskennung und eine messgenauigkeitsbezogene vierte Identifikationskennung zugeordnet werden. Dies hat zur Folge, dass dem Blutwertemessdatensatz, der aus der jeweiligen Blutprobe entstanden ist, zusätzlich neben der ersten und zweiten Identifikationskennung auch die dritte Identifikationskennung zur Angabe einer Identifikationsnummer des jeweiligen Messgerätes und die vierte Identifikationskennung zur Angabe des Grades einer Messgenauigkeit der angewendeten Messeinrichtung aufweist. Derartige Messeinrichtungen können beispielsweise datenelektronisch mit der Berechnungseinrichtung verbunden sein. Es ist denkbar, dass die Messeinrichtungen innerhalb eines Zentrallabors oder auf einer Station innerhalb eines Krankenhauses angeordnet sind. Alternativ kann natürlich nur eine einzige Messeinrichtung für einen Patienten verwendet werden, um mit dieser sämtliche Blutproben über einen gesamten Behandlungszeitraum zu messen.

Zusätzlich zu dem Zeitpunkt der Blutprobenentnahme kann auch der Zeitpunkt der Messung bestimmt werden, der ebenso als Identifikationskennung dem Blutwertemessdatensatz zugeordnet werden kann. Ebenso kann eine Identifikationskennung über die Klasse der Messeinrichtung, welche die Qualität der Messeinrichtung wiederspiegeln kann, sowie über weitere Qualitätsmerkmale der Messung an sich, zum Beispiel hinsichtlich der Genauigkeit, verwendet werden.

Somit umfasst ein Datenprotokoll von einer oder mehreren der Messeinrichtungen zu der Berechnungseinrichtung zumindest die Attribute der Patientenidentifikation, des Zeitpunktes der Messung, des Zeitpunktes der Probenentnahme, der Identifikation der Messeinrichtung, der Klasse der Messeinrichtung sowie ein Qualitätsmerkmal der Messung und den Messwert selbst. Gemäß einer bevorzugten Ausführungsform ist eine Vergleichseinheit innerhalb der Berechnungseinrichtung angeordnet, um die Blutwerte von verschiedenen Blutwertemessdaten bei einem Patienten zu vergleichen, und eine Differenzbestimmungseinheit innerhalb der Berechnungseinrichtung angeordnet, um die Differenz der verglichenen Blutwerte zu berechnen. Dies ermöglicht, dass nicht nur absolute Werte der gemessenen Blutwerte für die Berechnung der therapeutischen Daten verwendet werden, sondern auch Differenzwerte zu Blutwerten der vorausgegangenen gemessenen Blutproben oder auch Relationen zueinander, um so Langzeitanalysen des Blutwertespiegels als Grundlage für die als nächsten Schritt zu erfolgende Behandlung auf Grundlage der ermittelten therapeutischen Daten zu verwenden.

Eine Zuordnungseinheit innerhalb der Berechnungseinrichtung zum Zuordnen jedes Blutwertemessdatensatzes zu in einer Speichereinheit der Berechnungseinrichtung abgespeicherten patientenspezifischen Datensätze, die auch Blutwerte-Messdatensätze aus vorausgegangenen Messungen enthalten, kann ebenso angeordnet sein. Diese Zuordnungseinheit ermöglicht somit nach Überprüfung einer Patientenidentifikation (erste Identifikationskennung) bei Eingang des Blutwertemessdatensatzes in die Berechnungseinrichtung sowie nach einer Überprüfung der zweiten Identifikationskennung bezüglich des Zeitpunktes der Probenentnahme hinsichtlich Plausibilität die Zuordnung der eintreffenden Messdaten beziehungsweise des Blutwertemessdatensatzes zu einem patientenspezifischen Datensatz, der bereits abgespeichert ist. Dieser kann die Messergebnisse aus den vorangegangenen Blutwertemessdatensätzen dieses Patienten enthalten. Gegebenenfalls können weitere Daten, wie beispielsweise Besonderheiten des Patienten hinsichtlich seiner Anfälligkeit für bestimmte Medikamente enthalten sein. Vorrangig wird in diesem Datensatz der bisherige Verlauf der Blutwerte widergespiegelt.

Bei den Blutwerten kann es sich zum Beispiel um einen Glucosewert handeln, der als Referenz für die Dosierung des als Medikament zu verabreichenden Insulines dient.

Die Berechnungseinrichtung bewertet, wie bereits erwähnt, die betreffenden Blutwerte beziehungsweise den Blutwertemessdatensatz hinsichtlich des Blutproben-Entnahmezeitpunktes und der Messgenauigkeit der Messeinrichtung mit einem bestimmten Wertigkeitsfaktor unter Einbeziehung des bisherigen zeitlichen Blutwerteverlaufes. Es werden daraufhin Vorschläge zur Veränderung der Therapie errechnet und ebenso Vorschläge für die Verabreichung des Medikamentes und der daher spezifischen und vorher definierten relevanten Medikamentenparameter ausgegeben.

Ferner ist eine zweite Bewertungseinheit angeordnet, um jedem Blutwertmessdatensatz mit der gleichen ersten Identifikationserkennung und unterschiedlicher vierter Identifikationskennung mindestens einen individuellen Wertigkeitsfaktor, vorzugsweise einen Zahlenfaktor, zur Kennzeichnung einer Wertigkeit der Blutwertemessdaten in den Berechnungsvorgang zuzuordnen. Dies ermöglicht, dass die Messgenauigkeit der verminderten Messeinrichtung mit einem speziellen Zahlenfaktor bei der Berechnung der therapeutischen Daten berücksichtigt wird, sodass während des gesamten Behandlungsverlaufes von Blutprobe zu Blutprobe zwischen einzelnen Messeinrichtungen gewählt werden kann, auch wenn diese unterschiedliche Messgenauigkeiten aufweisen. Dies wird in dem sich anschließenden Berechnungsvorgang entsprechend berücksichtigt.

Vorzugsweise ist eine Messzeitpunktbestimmungseinheit zum Festlegen des Zeitpunktes der nächsten Messung unter Berücksichtigung der bisherigen Messdatensätze vorgesehen. Diese Messzeitpunktbestimmungseinrichtung kann somit nach der Berechnung der therapeutischen Daten und in Abhängigkeit von diesen therapeutischen Daten zugleich den Zeitpunkt der nächsten Messung beziehungsweise der nächsten Blutprobenentnahme aus dem Körper des Patienten festlegen, wobei diese Blutentnahmemessung auch automatisiert geschehen kann.

Vorteilhaft kann die Berechnungseinrichtung zusätzlich mit Informationen und Daten versehen werden, die von am Patienten angeschlossenen medizinischen Geräten gesendet werden. Dies kann beispielsweise die von einer Infusionspumpe verabreichten Medikamente und deren Medikamentenparameter sein, sodass bei dem Berechnungsvorgang auch die Medikamentenparameter und auch die Medikamente der momentanen Behandlung oder der zuvor vorausgegangenen Behandlung berücksichtigt werden.

Nach Ermittlung der therapeutischen Daten und der Erzeugung von Therapievorschlägen und der Messzeitpunkte werden diese zusammen mit der Patientenidentifikation, also der ersten Identifikationskennung, sowie mit der zweiten Identifikationskennung in digitaler Form an einer oder mehreren medizinischen Geräten, insbesondere Infusionspumpen übermittelt. Hierfür ist eine Zuführeinrichtung, vorzugsweise eine Insulinpumpe, angeordnet, um mindestens ein Medikament an den Patienten mit Medikamentenparametern, die von der Berechnungseinrichtung aus den therapeutisch bezogenen Daten ermittelt sind, zuzuführen.

Die Zuführeinrichtung, beziehungsweise die Insulinpumpe überprüft die patientenbezogene Identifikationskennung und die zeitpunktbezogene Identifikationskennung und aktiviert nach erfolgreicher Prüfung die Verabreichung von Medikamenten mit den aus den therapeutischen Daten ermittelten Medikamentenparametern gemäß der neu berechneten Therapie und den nächsten empfohlenen Messzeitpunkt. Zusätzlich oder alternativ können die therapeutischen Daten die Medikamentenparameter auch an der Zuführeinrichtung angezeigt werden.

Ein Verfahren zur Ermittlung von therapiebezogenen Daten für die Verabreichung mindestens eines Medikaments an einen zu therapierenden Patienten weist folgende Schritte auf:
- Fortlaufendes und aufeinanderfolgendes Entnehmen von Blutmengen von dem Patienten zum Erhalten einer Mehrzahl an Blutproben mittels mindestens einer Entnahmeeinrichtung
- Messen von Blutwerten der entnommenen Blutproben mittels mindestens einer, vorzugsweise mehrerer Blutwertemesseinrichtung(en)
   und Erhalten Blutwertemessdatensätze; und
- Berechnen von therapiebezogenen Daten aus den Blutwertemessdatensätzen und gegebenenfalls weiteren Daten mittels mindestens einer Berechnungseinrichtung,
   wobei jeder Blutprobe und jedem dazugehörigen Blutwertemessdatensatz zumindest eine patientenbezogene erste Identifikationskennung und eine zeitbezogene zweite Identifikationskennung über den Zeitpunkt der Entnahme der Blutprobe zugeordnet wird und die Berechnungseinrichtung für die Berechnung der therapiebezogenen Daten mittels einer ersten Bewertungseinheit jedem Blutwertemessdatensatz mit der gleichen ersten Identifikationskennung und unterschiedlicher zweiter Identifikationskennung mindestens einen individuellen Wertigkeitsfaktor, vorzugsweise einen Zahlenfaktor, zur Kennzeichnung einer Wertigkeit der Blutwertemessdaten in den Berechnungsvorgang zuordnet.

Mittels einer Zuordnungseinrichtung, die mit jeder Messeinrichtung verbunden ist, wird jedem Blutwertemessdatensatz eine der jeweiligen Messeinrichtung zugeordnete messgerätebezogene dritte Identifikationskennung und eine messgenauigkeitsbezogene vierte Identifikationskennung zugeordnet. Mittels einer zweiten Bewertungseinheit wird jedem Blutwertemessdatensatz mit der gleichen ersten Identifikationskennung und unterschiedlicher vierter Identifikationskennung mindestens ein individueller Wertigkeitsfaktor, vorzugsweise ein Zahlenfaktor, zur Kennzeichnung einer Wertigkeit der Blutwertemessdaten in dem Berechnungsvorgang zugeordnet.

Das ordnet mittels einer innerhalb der Berechnungseinrichtung angeordneten Zuordnungseinheit jeden Blutwertemessdatensatz zu in einer Speichereinheit der Berechnungseinrichtung abgespeicherten patientenspezifischen Datensätze, die auch Blutwertemessdatensätze aus vorausgegangenen Messungen enthalten.

Mittels einer Vergleichseinheit innerhalb der Berechnungseinrichtung werden die Blutwerte von verschiedenen Blutwertemessdatensätzen eines Patienten verglichen und mittels einer Differenzbestimmungseinheit innerhalb der Berechnungseinrichtung wird die Differenz der verglichenen Blutwerte berechnet.

Mittels einer Zuordnungseinrichtung, die mit jeder Messeinrichtung verbunden ist, wird jedem Blutwertemessdatensatz eine der jeweiligen Messeinrichtung zugeordnete messgerätebezogene dritte Identifikationskennung und eine messgenauigkeitsbezogene vierte Identifikationskennung zugeordnet. Weitere vorteilhafte Ausführungsformen ergeben sich aus den Unteransprüchen.

Vorteile und Zweckmäßigkeiten sind der nachfolgenden Beschreibung in Verbindung mit der Zeichnung zu entnehmen. Hierbei zeigen:
Fig. 1 in einer schematischen Darstellung ein Überblick über das erfindungsgemäße gesamte System;
Fig. 2 in einer schematischen Darstellung das erfindungsgemäße System mit detaillierten Angaben.

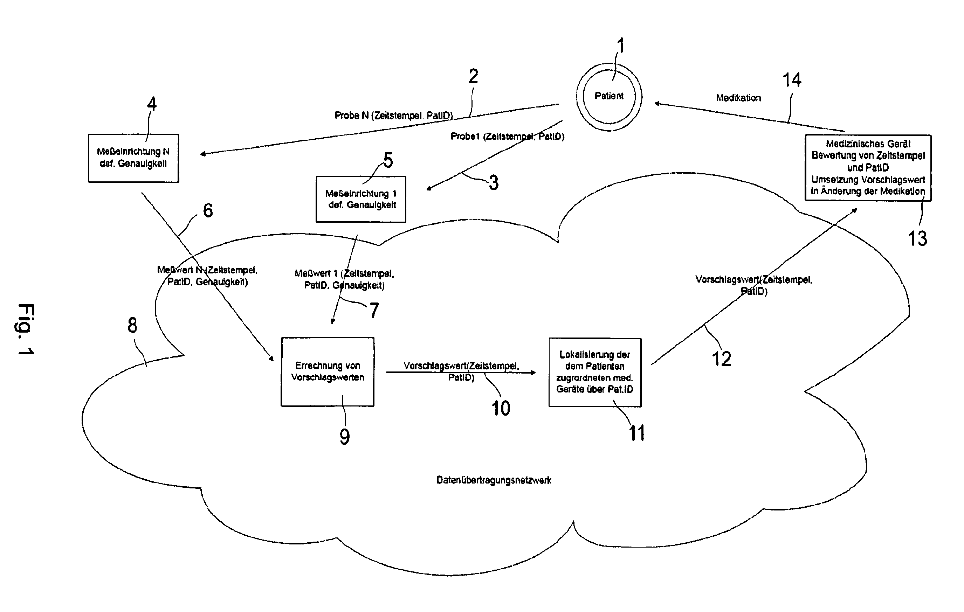

In Fig. 1 wird in einer schematischen Darstellung übersichtsweise das erfindungsgemäße System gemäß einer Ausführungsform der Erfindung dargestellt. Ein Patient 1 liegt beispielsweise auf einer Intensivstation eines Krankenhauses. Beliebig viele Blutproben N werden dem Patienten im Verlauf der Behandlungsdauer entnommen. Dies wird durch die beiden Bezugszeichen 2 und 3 wiedergegeben. Diese Blutproben erhalten nach der Entnahme einen Zeitstempel und eine Patientenidentifikation, welches der ersten und zweiten Identifikationskennung entspricht.

Die entnommenen Blutproben werden an N Messeinrichtungen 4, 5 übergeben, um Blutwertemessdaten zu messen. In diesem Fall sind zwei Messeinrichtungen 4, 5 mit einer vordefinierten Messgenauigkeit und einer Messeinrichtungs-Identifizierungsnummer abgebildet.

Nachdem die Messeinrichtungen die Blutwerte gemessen haben und eine jeweils einem Blutwertemessdatensatz erstellt haben, wird dieser Blutwertemessdatensatz mit dem von der jeweiligen Blutprobe herrührenden Zeitstempel der Patientenidentifizierungskennung und einer Identifikationskennung zu der Messgenauigkeit der Messeinrichtung und gegebenenfalls einer Identifikationskennung zu der einzelnen Messeinrichtung versehen. Dies wird dann, wie durch die Bezugszeichen 6 und 7 dargestellt an eine gemeinsame Berechnungseinrichtung 9 zum Berechnen von therapeutischen Daten als Vorschlagswerte weitergesendet.

Diese Berechnungseinrichtung 9 ist innerhalb eines gemeinsamen Datenübertragungsnetzwerkes 8 angeordnet, wobei die Übertragung der Messwerte vorzugsweise drahtlos wie beispielsweise per Wireless-LAN, oder auch drahtgebunden durchgeführt wird.

Innerhalb des Datenübertragungsnetzwerkes ist ebenso eine Art Lokalisierungseinrichtung 11 , die dazu dient, die jeweilige Infusionspumpe zur Verabreichung von Medikamenten für den spezifischen Patienten 1 , von dem die Blutprobe entnommen worden ist, zu orten. Hierfür kann die Lokalisierungseinrichtung 11 die mitgesendete Patientenidentifikationsnummer der ermittelten therapeutischen Daten, die auch weiterhin die Patientenidentifikationsnummer und einen Zeitstempel aufweisen, wie es durch das Bezugszeichen 10 gezeigt wird, benutzen.

Die therapeutischen Daten werden als Vorschlagswerte dann gemäß Bezugszeichen 12 zusammen mit der Patientenidentifikationsnummer und dem Zeitstempel an eine Infusionspumpe 13, die beispielsweise eine Insulinpumpe sein kann, gesendet.

Die Infusionspumpe 13 kann auch jede andere Art von medizinischem Gerät zur Verabreichung von Medikamenten sein.

Innerhalb dieser Infusionspumpe 13 findet nochmals eine Bewertung des Zeitstempels und der Patientenidentifikationsnummer statt, um festzustellen, dass die übermittelten therapeutischen Daten auch sicher als Medikation 14 diesem Patienten 1 zugeordnet sind. Ebenso findet die Umsetzung der therapeutischen Werte in eine spezielle Medikation, also beispielsweise Medikamentenparameter statt.

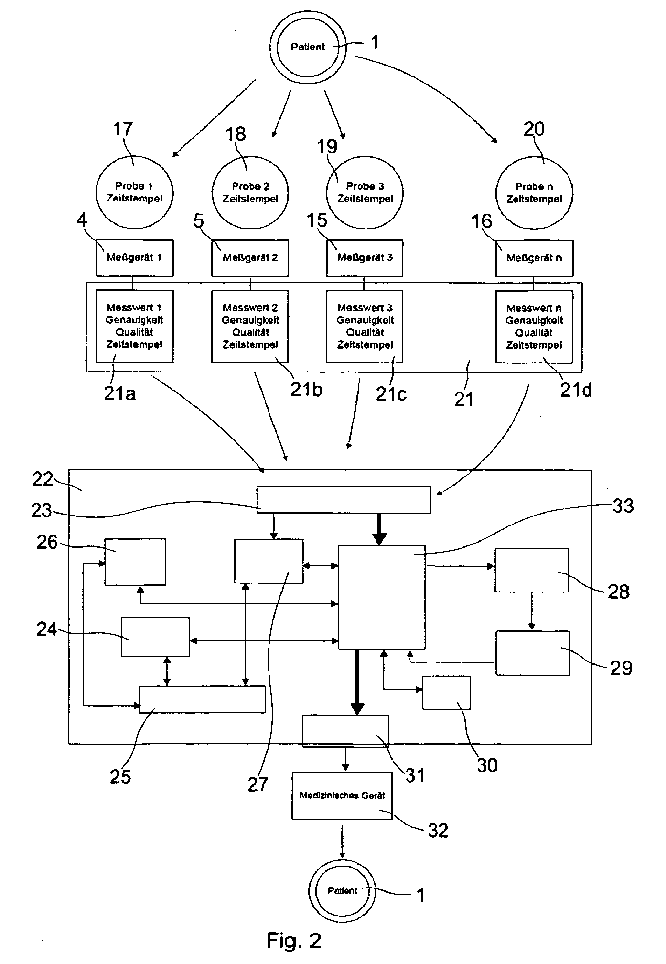

In Fig. 2 ist in einer schematischen Darstellung das erfindungsgemäße System mit näheren Details wiedergegeben. Gleiche und gleichbedeutende Teile sind zumindest teilweise mit gleichen Bezugszeichen versehen.

Ein Patient 1 erfährt nacheinander oder zeitgleich eine Blutentnahme mittels Blutentnahmeeinrichtungen 17, 18, 19 und 20.

Diese Blutentnahmeeinrichtungen ergeben eine Blutprobe, welche jeweils mit der Patientenidentifikationsnummer und einem Zeitstempel, der besagt, wann die Blutentnahme erfolgt ist, versehen wird.

Die Blutproben werden anschließend an Messeinrichtungen 4, 5, 15 und 16 mit teilweise unterschiedlichen Genauigkeitswerten in der Messung und mit unterschiedlichen Qualitätswerten gesendet. Diese Messgeräte 4, 5, 15 und 16 lassen sich zur Ermittlung von gleichen oder verschiedenen Blutwerten verwenden.

Bei Ermittlung von gleichen Blutwerten kann die Verwendung mehrerer Messeinrichtungen dazu dienen, dass die gemessenen Werte miteinander verglichen werden und somit eine Kontrolle der Richtigkeit der gemessenen Blutwerte stattfindet. Bei aufeinanderfolgender Messung der Blutwerte können die verschiedenen Messeinrichtungen dazu dienen, dass man dem Patienten an unterschiedlichen Orten hinsichtlich seiner Blutwerte messen kann.

Innerhalb einer Zuordnungseinrichtung 21 werden die jeweiligen Messwerte 21 a, 21 b, 21 c und 21d zusammen mit der Patientenidentifikationsnummer und dem Zeitstempel mit einer Identifikationskennung bezüglich der Messgenauigkeit der Messeinrichtungen und/oder der Qualität der Messeinrichtung sowie der Identifikationsnummer der Messeinrichtung zugeordnet. Anschließend wird in einer Berechnungseinrichtung 22 mittels einer Empfangseinheit 23 die Mehrzahl an Blutwertmessdaten, die von den Messeinrichtungen 4, 5, 15, 16 jeweils ermittelt und gemessen worden sind, empfangen.

Die Empfangseinheit 23 sendet sämtliche Blutmessdatensätze an eine zentral angeordnete Steuereinheit 33, die mittels einer ersten Bewertungseinheit 26 die Wertigkeit des Zeitstempels, also des Entnahmezeitpunktes, zu dem jeweiligen Blutwertemessdatensatz durchführt.

Die erste Bewertungseinheit 26 sendet dann einen entsprechenden Bewertungsfaktor an die Steuereinheit 33 zurück. Mittels einer zweiten Bewertungseinheit 27 wird die Wertigkeit derjenigen Messeinrichtung, die der Blutwertmessdatensatz geliefert hat, hinsichtlich ihrer Genauigkeit festgestellt. Sobald ein derartiger Wertigkeitsfaktor beziehungsweise Zahlenfaktor von der Bewertungseinheit 27 festgelegt worden ist, wird dieser der Steuereinheit 33 mitgeteilt.

Eine Zuordnungseinheit 24 ordnet die eingehenden Blutwertemessdatensätze nach Überprüfung der Patientenidentifikation und des Zeitstempels auf Plausibilität einem patienten-spezifischen Datensatz zu, der den bisherigen Behandlungsverlauf widerspiegelt und innerhalb einer Speichereinheit 25 abgespeichert ist. Dieser patientenbezogene Datensatz wird ebenso dafür hergenommen, um mittels der ersten Bewertungseinheit die Bewertungsfaktoren festzusetzen.

In einer Vergleichseinheit werden die Werte der vorausgegangenen Blutwertemessdatensätze mit den Blutwerten der aktuellen Blutwertemessdatensatze verglichen, um gegebenenfalls Differenzbeträge gemäß einer Differenzbestimmungseinheit 29 zwischen diesen Blutwerten festzustellen. Festgestellte Differenzen werden zur Berücksichtigung von derartigen Differenzen und zur Berechnung von Relationen an die Steuereinheit 33 zurückgesendet, um nicht nur mit absoluten Blutwerten sondern auch mit Relationen und Differenzen zu den gemessenen Vorgängerblutwerten bei der Berechnung zu arbeiten.

In einer Mess- und Bestimmungseinheit 30 wird dann auch nach erfolgter Berechnung der therapeutischen Daten der Zeitpunkt der nächsten Messung bestimmt und in Form einer Anzeige empfohlen.

Sämtliche Daten, die dann als therapeutische Daten nach erfolgter Berechnung vorliegen, werden an eine gemeinsame Sendeeinheit 31 von Seiten der Steuereinheit 33 übertragen, um dann anschließend diese therapeutischen Daten an ein medizinisches Gerät 32, welches eine Insulinpumpe sein kann, zu senden. Die Insulinpumpe ermittelt aus den therapeutischen Daten unter anderem Medikamentenparameter für die Verabreichung des Insulins, um anschließend dem Patienten 1 das Insulin in Form der empfohlenen Medikamentation zu verabreichen.

### Bezugszeichenliste

- 1: Patient
- 2: Blutprobe
- 3: Blutprobe
- 4: Blutwertemesseinrichtung
- 5: Blutwertemesseinrichtung
- 6: Blutwertemessdatensätze
- 7: Blutwertemessdatensätze
- 8: Datenübertragungsnetzwerk
- 9: Berechnungseinrichtung
- 10: Vorschlagswert
- 11: Lokalisierungseinrichtung
- 12: Vorschlagswert
- 13: Zuführeinrichtung
- 14: Medikament
- 15: Blutwertemesseinrichtung
- 16: Blutwertemesseinrichtung
- 17: Entnahmeeinrichtung
- 18: Entnahmeeinrichtung
- 19: Entnahmeeinrichtung
- 20: Entnahmeeinrichtung
- 21: Zuordnungseinrichtung
- 21 a-d: Blutwertemessdatensätze
- 22: Berechnungseinrichtung
- 23: Empfangseinheit
- 24: Zuordnungseinheit
- 25: Speichereinheit
- 26: Bewertungseinheit
- 27: Bewertungseinheit
- 28: Vergleichseinheit
- 29: Differenzbestimmungseinheit
- 30: Messzeitpunkt-Bestimmungseinheit
- 31: Sendeeinheit
- 32: Zuführeinrichtung
- 33: Steuereinheit

## Patentansprüche

1. System zur Ermittlung von therapiebezogenen Daten für die Verabreichung von mindestens einem Medikament an einen zu therapierenden Patienten (1), umfassend:
- Mindestens eine Entnahmeeinrichtung (17-20), um fortlaufend und aufeinanderfolgend Blutmengen von dem Patienten (1) zum Erhalten einer Mehrzahl an Blutproben (2, 3; 17-20) zu entnehmen;
- mehrere Blutwertemesseinrichtungen (4, 5; 15, 16), um Blutwerte der entnommenen Blutproben (2, 3; 17 - 20) zu messen und Blutwertemessdatensätze (6, 7; 21 a-d) zu erhalten; und
- mindestens eine Berechnungseinrichtung (22; 9), um therapiebezogene Daten aus den Blutwertemessdatensätzen (6, 7; 21a-d) und gegebenenfalls weiteren Daten zu berechnen; wobei
jeder Blutprobe (2, 3; 17-20) und jedem dazugehörigen Blutwertemessdatensatz (6, 7; 21a-d) zumindest eine patientenbezogene erste Identifikationskennung und eine zeitbezogene zweite Identifikationskennung über den Zeitpunkt der Entnahme der Blutprobe (2, 3; 17-20) zugeordnet ist;
**dadurch gekennzeichnet, dass**
die Berechnungseinrichtung (22; 9) für die Berechnung der therapiebezogenen Daten mittels einer ersten Bewertungseinheit (26) jedem Blutwertemessdatensatz (6, 7; 21 a-d) mit der gleichen ersten Identifikationskennung und unterschiedlicher zweiter Identifikationskennung mindestens einen individuellen Wertigkeitsfaktor, vorzugsweise einen Zahlenfaktor, zur Kennzeichnung einer Wertigkeit der Blutwertemessdaten (6, 7; 21a-d) in den Berechnungsvorgang zuordnet;
eine Zuordnungseinrichtung (21) vorgesehen ist, die mit jeder Blutwertmesseinrichtung (4, 5, 15, 16) verbunden ist, um jedem Blutwertemessdatensatz (21a-d) eine der jeweiligen Blutwertemesseinrichtung (4, 5,15, 16) zugeordnete messgerätebezogene dritte Identifikationskennung und eine messgenauigkeitsbezogene vierte Identifikationskennung zuzuordnen; und
eine zweite Bewertungseinheit (27) vorgesehen ist, um jeden Blutwertemessdatensatz (21 a-d) mit dergleichen ersten Identifikationskennung und unterschiedlicher vierter Identifikationskennung mindestens einen individuellen Wertigkeitsfaktor, vorzugsweise einen Zahlenfaktor, zur Kennzeichnung einer Wertigkeit der Blutwertemessdaten in den Berechnungsvorgang zuzuordnen.

2. System nach Anspruch 1, ferner mit einer Vergleichseinheit (28) innerhalb der Berechnungseinrichtung (22), um die Blutwerte von verschiedenen Blutwertemessdatensätzen (6, 7; 21a-d) eines Patienten (1) zu vergleichen, und eine Differenzbestimmungseinheit (29) innerhalb der Berechnungseinrichtung (22) zum Berechnen der Differenz der verglichenen Blutwerte.

3. System nach Anspruch 1 oder 2, ferner mit einer Zuordnungseinheit (24) innerhalb der Berechnungseinrichtung (22) zum Zuordnen jedes Blutwertemessdatensatzes (6, 7; 21 a-d) zu in einer Speichereinheit (25) der Berechnungseinrichtung (22) abgespeicherten patientenspezifischen Datensätze, die auch Blutwertemessdatensätze aus vorausgegangenen Messungen enthalten.

4. System nach einem der vorangegangenen Ansprüche, ferner mit einer Messzeitpunkt-Bestimmungseinheit (30) zum Festlegen des Zeitpunktes der nächsten Messung unter Berücksichtigung der bisherigen Blutwertemessdatensätze (6, 7; 21 a-d).

5. System nach einem der vorangegangenen Ansprüche, ferner mit einer Zuführeinrichtung (13; 32), vorzugsweise eine Insulinpumpe, zum Zuführen von mindestens einem Medikament (14) an den Patienten (1) mit Medikamentenparametern, die von der Berechnungseinrichtung (22) aus den therapiebezogenen Daten ermittelt sind.

6. Verfahren zur Ermittlung von therapiebezogenen Daten für die Verabreichung von mindestens einem Medikament an einen zu therapierenden Patienten (1), mit folgenden Schritten:
- Fortlaufendes und aufeinanderfolgendes Entnehmen von Blutmengen von dem Patienten (1) zum Erhalten einer Mehrzahl an Blutproben (2, 3; 17-20) mittels mindestens einer Entnahmeeinrichtung (17-20);
- Messen von Blutwerten der entnommenen Blutproben (2, 3; 17-20) mittels mehrerer Blutwertemesseinrichtungen (4, 5, 15,16) und Erhalten von Blutwertemessdatensatze (6, 7; 21 a-d); und
- Berechnen von therapiebezogenen Daten aus den Blutwertemessdatensätzen (6, 7; 21 a-d) und gegebenenfalls weiteren Daten mittels mindestens einer Berechnungseinrichtung (22; 9), wobei
jeder Blutprobe (2, 3; 17-20) und jedem dazugehörigen Blutwertemessdatensatz (6, 7; 21 a-d) zumindest eine patientenbezogene erste Identifikationskennung und eine zeitbezogene zweite Identifikationskennung über den Zeitpunkt der Entnahme der Blutprobe (2, 3; 17-20) zugeordnet wird,
**dadurch gekennzeichnet, dass**
die Berechnungseinrichtung (22; 9) für die Berechnung der therapiebezogenen Daten mittels einer ersten Bewertungseinheit (26) jedem Blutwertemessdatensatz (6, 7; 21 a-d) mit der gleichen ersten Identifikationskennung und unterschiedlicher zweiter Identifikationskennung mindestens einen individuellen Wertigkeitsfaktor, vorzugsweise einen Zahlenfaktor, zur Kennzeichnung einer Wertigkeit der Blutwertemessdaten (6, 7; 21a-d) in dem Berechnungsvorgang zuordnet;
mittels einer Zuordnungseinrichtung (21), die mit jeder Blutwertemesseinrichtung (4, 5, 15, 16) verbunden ist, jedem Blutwertemessdatensatz (21a-d) eine der jeweiligen Blutwertemesseinrichtung (4, 5, 15, 16) zugeordnete messgerätebezogene dritte Identifikationskennung und eine messgenauigkeitsbezogene vierte Identifikationskennung zugeordnet wird, und
mittels einer zweiten Bewertungseinheit (27) jedem Blutwertemessdatensatz (21 a-d) mit der gleichen ersten Identifikationskennung und unterschiedlicher vierter Identifikationskennung mindestens ein individueller Wertigkeitsfaktor, vorzugsweise ein Zahlenfaktor, zur Kennzeichnung einer Wertigkeit der Blutwertemessdaten in dem Berechnungsvorgang zugeordnet wird.

7. Verfahren nach Anspruch 6, wobei mittels einer innerhalb der Berechnungseinrichtung (22) angeordneten Zuordnungseinheit (24) jeder Blutwertemessdatensatz (6, 7; 21 a-d) zu in einer Speichereinheit (25) der Berechnungseinrichtung (22) abgespeicherten patientenspezifischen Datensätze, die auch Blutwertemessdatensatze aus vorausgegangenen Messungen enthalten, zugeordnet wird.

8. Verfahren nach Anspruch 6 oder 7, wobei mittels einer Vergleichseinheit (28) innerhalb der Berechnungseinrichtung (22) die Blutwerte von verschiedenen Blutwertemessdatensätzen (6, 7; 21a-d) eines Patienten (1) verglichen werden und mittels einer Differenzbestimmungseinheit (29) innerhalb der Berechnungseinrichtung (22) die Differenz der verglichenen Blutwerte berechnet werden.

## Claims

1. A system for determining treatment-related data for the administration of at least one drug to a patient (1) to be treated, comprising:
- at least one sampling device (17-20) for continuously and successively taking quantities of blood from the patient (1) in order to obtain a plurality of blood samples (2, 3; 17-20);
- multiple blood value measuring devices (4, 5, 15, 16) in order to measure blood values of the blood samples (2, 3; 17-20) taken and to obtain blood value measurement data sets (6, 7; 21 a-d); and
- at least one calculating device (22; 9) for calculating the treatment-related data from the blood value measurement data sets (6, 7; 21 a-d) and, if appropriate, further data, wherein
at least one patient-related first identifier and one time-related second identifier concerning the point of time of taking the blood sample (2, 3; 17-20) is assigned to each blood sample (2, 3; 17-20) and to each blood value measurement data set (6, 7; 21a-d) associated therewith,
**characterized in that**
by means of a first evaluation unit (26), the calculating device (22; 9) for calculation of the treatment-related data assigns at least one individual weighting factor, preferably a numerical factor, to each blood value measurement data set (6, 7; 21 a-d) with the same first identifier and a different second identifier in order to characterize a weighting of the blood value measurement data (6, 7; 21a-d) in the calculation operation;
an assigning device (21) is provided which is connected to each blood value measuring device (4, 5, 15, 16) in order to assign a third identifier related to the measuring equipment, being assigned to the particular blood value measuring devices (4, 5, 15, 16), and a fourth identifier related to the measurement accuracy to each blood value measurement data set (6, 7; 21a-d); and
a second evaluation unit (27) is provided in order to assign at least one individual weighting factor, preferably a numerical factor, for characterizing a weighting of the blood value measurement data in the calculation operation to each blood value measurement data set (6, 7; 21 a-d) with the same first identifier and a different fourth identifier.

2. The system as claimed in claim 1, further comprising a comparison unit (28) disposed within the calculating device (22) in order to compare the blood values of different blood value measurement data sets (6, 7; 21 a-d) of a patient (1), and a differentiation unit (29) disposed within the calculating device (22; 9) in order to calculate the difference between the compared blood values.

3. The system as claimed in claim 1 or 2, further comprising an assigning unit (24) disposed within the calculating device (22) for assignment of each blood value measurement data set (6, 7; 21 a-d) to patient-specific data sets which are stored in a storage unit (25) of the calculating device (22) and which also include blood value measurement data sets (6, 7; 21a-d) from preceding measurements.

4. The system as claimed in one of the preceding claims, further comprising a measurement time determining unit (30) for fixing the point of time of the next measurement taking into account of the previous blood value measurement data sets (6, 7; 21 a-d).

5. The system as claimed in one of the preceding claims, further comprising a delivery device (13; 32), preferably an insulin pump, to deliver at least one drug (14) to the patient (1) with drug parameters which are determined from the treatment-related data by the calculating device (22).

6. A method for determining treatment-related data for the administration of at least one drug to a patient (1) to be treated, comprising the following steps:
- continuously and successively taking quantities of blood from the patient (1) in order to obtain a plurality of blood samples (2, 3; 17-20) by means of at least one sampling device (17-20);
- measuring blood values of the blood samples (2, 3; 17-20) taken by means of multiple blood value measuring devices (4, 5, 15, 16) and obtaining blood value measurement data sets (6, 7; 21a-d); and
- calculating the treatment-related data from the blood value measurement data sets (6, 7; 21 a-d) and, if appropriate, further data by means of at least one calculating device (22; 9), wherein
at least one patient-related first identifier and one time-related second identifier concerning the point of time of taking the blood sample (2, 3; 17-20) are assigned to each blood sample (2, 3; 17-20) and each blood value measurement data set (6, 7; 21 a-d) associated therewith,
**characterized in that**,
by means of a first evaluation unit (26), the calculating device (22; 9) for calculation of the treatment-related data assigns at least one individual weighting factor, preferably a numerical factor, to each blood value measurement data set (6, 7; 21 a-d) with the same first identifier and a different second identifier in order to characterize a weighting of the blood value measurement data in the calculation operation;
by means of an assigning device (21), which is connected to each measuring device (4, 5, 15, 16), a third identifier related to the measuring equipment and assigned to the particular measuring devices (4, 5, 15, 16) and a fourth identifier related to the measurement accuracy is assigned to each blood value measurement data set (21a-d), and
by means of a second evaluation unit (27), at least one individual weighting factor, preferably a numerical factor, is assigned to each blood value measurement data set (6, 7; 21 a-d) with the same first identifier and a different fourth identifier in order to characterize a weighting of the blood value measurement data in the calculation operation.

7. The method as claimed in claim 6, wherein, by means of an assigning unit (24) disposed within the calculating device (22), patient-specific data sets which are stored in a storage unit (25) of the calculating device (22) and which also include blood value measurement data sets (6, 7; 21a-d) from preceding measurements are assigned to each blood value measurement data set (6, 7; 21a-d).

8. The method as claimed in claim 6 or 7, wherein, by means of a comparison unit (28) disposed within the calculating device (22), the blood values of different blood value measurement data sets (6, 7; 21a-d) of a patient (1) are compared, and, by means of a differentiation unit (29) disposed within the calculating device (22), the difference between the compared blood values is calculated.

## Revendications

1. Système de détermination de données relatives à un traitement pour l'administration d'au moins un médicament à un patient devant recevoir un traitement (1), comprenant :
- au moins un dispositif de prélèvement (17-20) pour prélever de façon permanente et successive des doses de sang du patient (1) afin d'obtenir une pluralité d'échantillons de sang (2, 3 ; 17-20) ;
- plusieurs dispositifs de mesure des valeurs sanguines (4, 5 ; 15, 16) pour mesurer les valeurs sanguines des échantillons de sang (2, 3 ; 17-20) prélevés et obtenir des ensembles de données de mesure sur les valeurs sanguines (6, 7 ; 21a-d) ; et
- au moins un dispositif de calcul (22 ; 9) pour calculer des données relatives à un traitement à partir des ensembles de données de mesure sur les valeurs sanguines (6, 7 ; 21 a-d) et, le cas échéant, d'autres données ; étant entendu qu'au moins une première caractéristique d'identification liée au patient et une deuxième caractéristique d'identification liée au temps et relatives au moment du prélèvement de l'échantillon de sang (2, 3; 17-20) sont attribuées à chaque échantillon de sang (2, 3 ; 17-20) et à chaque ensemble de données de mesure sur les valeurs sanguines (6, 7 ; 21 a-d) y afférent ;
**caractérisé en ce que**
le dispositif de calcul (22 ; 9) destiné à calculer les données relatives à un traitement attribue au moins un facteur de valence individuel, de préférence un facteur numérique, à chaque ensemble de données de mesure sur les valeurs sanguines (6, 7 ; 21a-d) ayant la même première caractéristique d'identification et une deuxième caractéristique d'identification différente, au moyen d'une première unité d'analyse (26), afin de caractériser une valence des données de mesure sur les valeurs sanguines (6, 7 ; 21 a-d) dans le processus de calcul ;
il est prévu un dispositif d'attribution (21) qui est relié à chaque dispositif de mesure des valeurs sanguines (4, 5, 15, 16) afin d'attribuer à chaque ensemble de données de mesure des valeurs sanguines (21 a-d) une troisième caractéristique d'identification liée à l'appareil de mesure attribuée au dispositif de mesure des valeurs sanguines (4, 5, 15, 16) correspondant et une quatrième caractéristique d'identification liée à la précision de mesure ; et
il est prévu une deuxième unité d'analyse (27) pour attribuer au moins un facteur de valence individuel, de préférence un facteur numérique, à chaque ensemble de données de mesure sur les valeurs sanguines (21 a-d) ayant la même première caractéristique d'identification et une quatrième caractéristique d'identification différente, afin de caractériser une valence des données de mesure sur les valeurs sanguines dans le processus de calcul.

2. Système selon la revendication 1, comprenant également une unité de comparaison (28), à l'intérieur du dispositif de calcul (22), destinée à comparer les valeurs sanguines de différents ensembles de données de mesures sur les valeurs sanguines (6, 7; 21a-d) d'un patient (1), et une unité de détermination de différence (29), à l'intérieur du dispositif de calcul (22), destinée à calculer la différence des valeurs sanguines comparées.

3. Système selon la revendication 1 ou 2, comprenant également une unité d'attribution (24), à l'intérieur du dispositif de calcul (22), destinée à attribuer chaque ensemble de données de mesure sur les valeurs sanguines (6, 7 ; 21 a-d) à des ensembles de données spécifiques au patient enregistrés dans une unité de mémoire (25) du dispositif de calcul (22) et qui comprennent également des ensembles de données de mesure sur les valeurs sanguines provenant de mesures précédentes.

4. Système selon l'une des revendications précédentes, comprenant également une unité de détermination de moment de mesure (30) destinée à déterminer le moment de la mesure suivante en prenant en considération les ensembles déjà existants de données de mesure sur les valeurs sanguines (6, 7 ; 21a-d).

5. Système selon l'une des revendications précédentes, comprenant également un dispositif de délivrance (13 ; 32), de préférence une pompe à insuline, pour délivrer au moins un médicament (14) au patient (1) en fonction de paramètres de médicament qui sont déterminés par le dispositif de calcul (22) à partir des données relatives au traitement.

6. Procédé de détermination de données relatives à un traitement pour l'administration d'au moins un médicament à un patient devant recevoir un traitement (1), comprenant les étapes suivantes :
- prélèvement de façon permanente et successive de doses de sang du patient (1) afin d'obtenir une pluralité d'échantillons de sang (2, 3 ; 17-20) au moyen d'au moins un dispositif de prélèvement (17-20) ;
- mesure de valeurs sanguines des échantillons de sang (2, 3 ; 17-20) prélevés au moyen de plusieurs dispositifs de mesure des valeurs sanguines (4, 5, 15, 16) et obtention d'ensembles de données de mesure sur les valeurs sanguines (6, 7 ; 21a-d) ; et
- calcul de données relatives à un traitement à partir des ensembles de données de mesure sur les valeurs sanguines (6, 7 ; 21a-d) et, le cas échéant, d'autres données au moyen d'un dispositif de calcul (22 ; 9) ;
au moins une première caractéristique d'identification liée au patient et une deuxième caractéristique d'identification liée au temps et relative au moment du prélèvement de l'échantillon de sang (2, 3; 17-20) étant attribuées à chaque échantillon de sang (2, 3 ; 17-20) et à chaque ensemble de données de mesure sur les valeurs sanguines (6, 7 ; 21 a-d) y afférent;
**caractérisé en ce que**
le dispositif de calcul (22 ; 9) destiné à calculer les données relatives à un traitement attribue au moins un facteur de valence individuel, de préférence un facteur numérique, à chaque ensemble de données de mesure sur les valeurs sanguines (6, 7 ; 21 a-d) ayant la même première caractéristique d'identification et une deuxième caractéristique d'identification différente, au moyen d'une première unité d'analyse (26), afin de caractériser une valence des données de mesure sur les valeurs sanguines (6, 7 ; 21a-d) dans le processus de calcul ;
une troisième caractéristique d'identification liée à l'appareil de mesure et se rapportant au dispositif de mesure des valeurs sanguines (4, 5, 15, 16) correspondant et une quatrième caractéristique d'identification liée à la précision de mesure sont attribuées à chaque ensemble de données de mesure des valeurs sanguines (21 a-d) au moyen d'un dispositif d'attribution (21) qui est relié avec chaque dispositif de mesure des valeurs sanguines (4, 5, 15, 16) ; et
au moins un facteur de valence individuel, de préférence un facteur numérique, est attribué à chaque ensemble de données de mesure sur les valeurs sanguines (21 a-d) ayant la même première caractéristique d'identification et une quatrième caractéristique d'identification différente, au moyen d'une deuxième unité d'analyse (27), afin de caractériser une valence des données de mesure sur les valeurs sanguines dans le processus de calcul.

7. Procédé selon la revendication 6, dans lequel chaque ensemble de données de mesure sur les valeurs sanguines (6, 7 ; 21a-d) est attribué, au moyen d'une unité d'attribution (24) agencée à l'intérieur de l'unité de calcul (22), à des ensembles de données spécifiques au patient enregistrés dans une unité de mémoire (25) du dispositif de calcul (22), qui comprennent également des ensembles de données de mesure sur les valeurs sanguines provenant de mesures précédentes.

8. Procédé selon la revendication 6 ou 7, dans lequel les valeurs sanguines de différents ensembles de données de mesures sur les valeurs sanguines (6, 7 ; 21 ad) d'un patient (1) sont comparées au moyen d'une une unité de comparaison (28) à l'intérieur du dispositif de calcul (22) et la différence des valeurs sanguines comparées est calculée au moyen d'une unité de détermination de différence (29) à l'intérieur du dispositif de calcul (22).
